# EUROPEAN PATENT APPLICATION

(11) **EP 1 836 981 A2**
(43) Date of publication of application: **26.09.2007**
(21) Application number: 07251191.8
(22) Date of filing: 21.03.2007
(51) Int. Cl.: A61B 17/68

(54) **A bone fracture fixation system**

(30) Priority: 24.03.2006 US 388264
(71) Applicant: DePuy Products, Inc., Warsaw, IN 46581 (US)
(72) Inventor: Deffenbaugh, Daren L., Winona Lake, IN 46590 (US); Schulze, Dale R., Springboro, OH 45066 (US)
(74) Representative: Belcher, Simon James

(57) **Abstract**

A bone fracture fixation system comprises a bone plate (10) configured to bear against a proximal surface (P) of the bone and a plurality of elongated tension elements (22), each sized to pass through an opening (14a) in the bone plate and through the bone from the proximal surface to a distal surface (D) thereof. Each tension element is anchored to the bone and maintained in tension by a distal anchor (24) attached to the tension element and configured to engage the distal surface of the bone and a proximal anchor (26) engageable between the bone plate and the tension element.

## Description

The present invention relates to a bone fracture fixation system, in particular which can be used for internal fixation of fractures of the bone.

For any bone fracture, the orthopaedic specialist must first reduce the fracture and then adequately stabilize and fix the bone to maintain the reduction as the bone heals. Each of these steps is complicated when the bone has suffered multiple fractures or a fracture that is not simply transverse. Fractures of this type usually require some form of internal fixation to reduce and maintain the bone fragments. One conventional approach to reducing bone fragments is with a bone plate extending over a portion of the bone at the fracture site. In one surgical approach, an orthopaedic surgeon may use a specialized clamp applied across the bone while attaching the fixation plate to the bone by screwing a plurality of screw fasteners through holes in the plate into the underlying bone.

Generally, screw fasteners are effective in holding the bone plate tightly against the healthy bone so that fracture may heal properly. However, in less healthy (e.g., osteopenic) bone, the screw threads may not find adequate purchase in the bone to hold the bone and plate together in proper alignment. This may result in non-union of the fracture that may require more invasive revision surgery to correct.

Another problem associated with screw fasteners is occasional breakage of the screw near the plate-bone interface due to stress concentrations arising from poor load sharing among all of the screws and/or high cyclic loading.

A further drawback of the screw fastener approach to internal fixation is that a large variety of screw sizes must be made available for each surgical procedure in order to accommodate variations in patient anatomy and fracture type. Maintaining a large inventory of screw sizes, along with the appropriately sized drills, guides, drivers and fixtures, can be costly. There is also the chance that an inappropriately sized screw may be selected during a procedure.

Fixation approaches have been developed that do not rely upon screw fasteners. For example, cerclage systems utilize one or more cables tightened around a bone to hold the fracture fragments together. The cable construct may include a plate that helps anchor the cables. However, cerclage systems require access around the entire periphery of the bone so it is necessary for the surgeon to dissect soft tissues surrounding the bone. Another problem is that the cerclage cable can exert significant line pressure against the periosteum, which may injure the bone and inhibit healing.

Accordingly, there is a need for a fracture fixation system that provides a stable construct in osteopenic bone and that may be adapted for minimally invasive surgical procedures. There is also a need for a fracture fixation system that reduces the inventory of fasteners and associated instrumentation required during the surgical procedure. There is a further need for a fracture fixation system that assists in improving and maintaining the fracture reduction during application and that incorporates fasteners that resist breakage after implantation and that are less technique sensitive to apply than conventional bone screws.

The present invention provides a fracture fixation system that comprises a bone plate configured to bear against a proximal surface of the bone, the bone plate having a plurality of openings extending through it. A plurality of elongated tension elements are provided, each sized to pass through one of the bone plate openings and through the bone from the proximal surface to a distal surface thereof. A distal anchor is attached to each tension element and configured to engage the distal surface of the bone when the tension element passes through the bone. A proximal anchor can be deployed between the bone plate and each tension element to maintain tension in the tension element between the proximal anchor and the distal anchor. The tension element may be a braided metal cable or similar elongated element.

In one embodiment, the proximal anchor is a Tinnerman washer. In this embodiment, the bone plate defines a recess around at least some of the plurality of openings. The Tinnerman washer is sized to be received within the recess and is configured to engage the tension element passing through it to maintain tension on the element.

In other embodiments, the proximal anchor is configured for a polyaxial interface with the bone plate. With this feature, the tension element may be situated at a range of angles relative to the bone plate to optimize the ability of the surgeon to reduce multiple bone fracture fragments. In these embodiments, at least some of the openings define a spherical wall and include a locking bushing disposed therein. The locking bushing has a spherical outer surface for complementary engagement with the spherical wall, and further includes an internally threaded bore. The proximal anchor includes a central bore for receiving the tension element through it and a threaded head. The head of the anchor and the locking bushing define a tapered threaded interface so that the bushing expands into the spherical wall as the head is threaded into the bushing.

In one aspect, the distal anchor has a shape memory component with a first configuration sized to pass through the bone plate openings and through the bone from the proximal surface to a distal surface thereof. The shape memory component has a second shape memory configuration for engaging the distal surface of the bone. In one embodiment, the distal anchor includes at least two prongs that are substantially aligned with the elongated tension element in the first configuration and extended outward therefrom in the second shape memory configuration. The prongs may be spring elements or may be formed of a shape memory metal.

In other embodiments, the distal anchor is a fixed shape component that is sized to bear against the distal surface of the bone. Thus, the distal anchor may be a disc or a generally spherical element. In specific embodiments, the distal anchor may also include a washer with a recess to receive the spherical element therein.

The system of the present invention can be used in a method for fixation of a bone fracture that comprises positioning a bone plate on a proximal surface of the bone, the bone plate including a plurality of openings through it and passing at least two elongated tension elements through plate openings and through the bone to a distal surface thereof. Each tension elements is anchored to the distal surface of the bone and then placed in tension. The proximal end of the tension element is then anchored to the bone plate while maintaining the tension in the element. To facilitate passage of each tension element through the bone, a K-wire may be used to form an appropriately oriented path and a sheath may be used to help convey the tension element, and in some cases the distal anchor, through the bone.

One significant benefit of the fracture fixation system of the present invention is that it is well suited for use in soft, osteopenic bone that is otherwise incapable of supporting a lag screw. At the same time, the tension elements provide a lag effect for optimal fracture reduction.

Another benefit is that the tension elements and anchors may be provided as "one size fits all". In other words, once the tension elements are tensioned and anchored, any excess material is removed. The necessary size of the tension element need not be determined prior to implantation. The tension element may be arranged to pass directly across the bone or across long spans of the bone without requiring differently sized elements.

The fracture fixation system of the invention can be used to reduce multiple fracture fragments. It can be used with healthy or less than healthy bone.

Embodiments of the invention will now be described by way of example with reference to the accompanying drawings, in which:
FIG. 1 is a side perspective of a fracture fixation system according to one embodiment of the present invention.
FIG. 2 is an enlarged cross-sectional view of the fracture fixation system shown in FIG. 1.
FIG. 3 is an enlarged cross-sectional view of a fracture fixation system according to a further embodiment of the invention.
FIG. 4 is an enlarged perspective view of a proximal anchor used in the fracture fixation system shown in FIG. 3.
FIG. 5 is an enlarged perspective partial view of the working end of a driving tool for driving the proximal anchor shown in FIG. 4.
FIGS. 6a, 6b are side views of a distal anchor for use with the fracture fixation system shown in FIG. 1 according to one embodiment of the invention, with the distal anchor shown in its non-extended and extended states.
FIGS. 7a, 7b are side partial cross-sectional views of the distal anchor depicted in FIGS. 6a, 6b, shown with the distal anchor within a bone in its extended and tensioned states.
FIGS. 8a, 8b are side partial cross-sectional and bottom views of a distal anchor for use with the fracture fixation system shown in FIG. 1, according to a further embodiment of the invention.
FIG. 9 is a side perspective view of a distal anchor for use with the fracture fixation system shown in FIG. 1, according to still another embodiment of the invention.
FIGS. 10a, 10b are side views of additional distal anchors of a distal anchor for use with the fracture fixation system shown in FIG. 1.
FIG. 11a is a side view of a tension element and distal anchor for use with the fracture fixation system shown in FIG. 1, according to one embodiment of the invention.

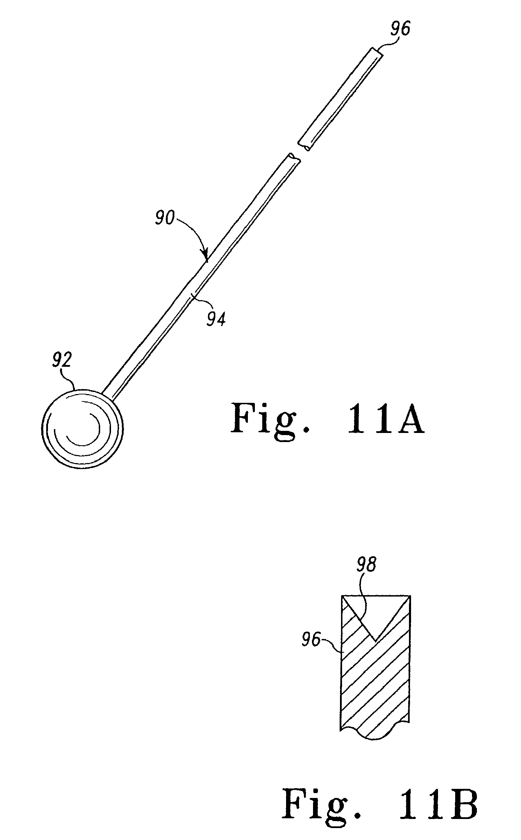

FIG. 11 b is an enlarged partial cross-sectional view of the proximal end of the tension element shown in FIG. 11a.

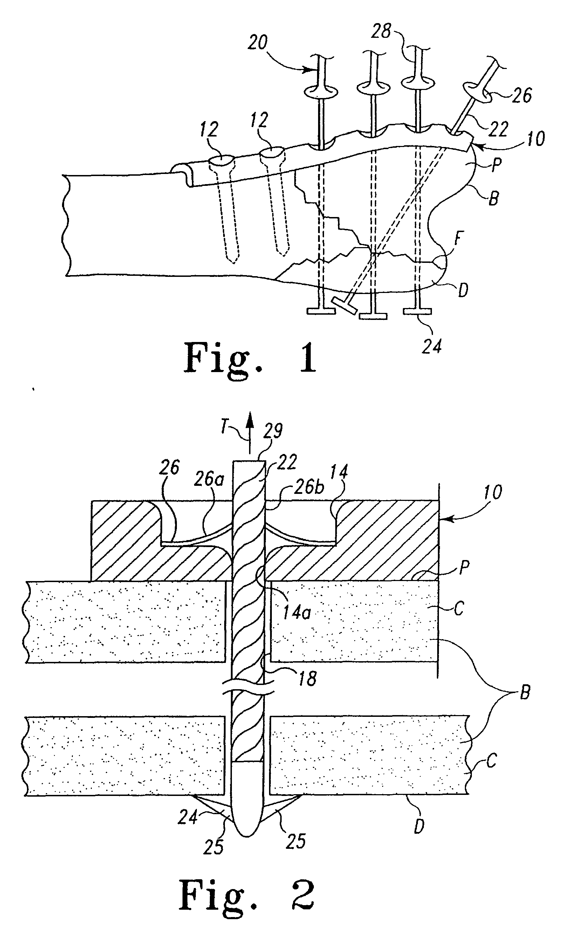

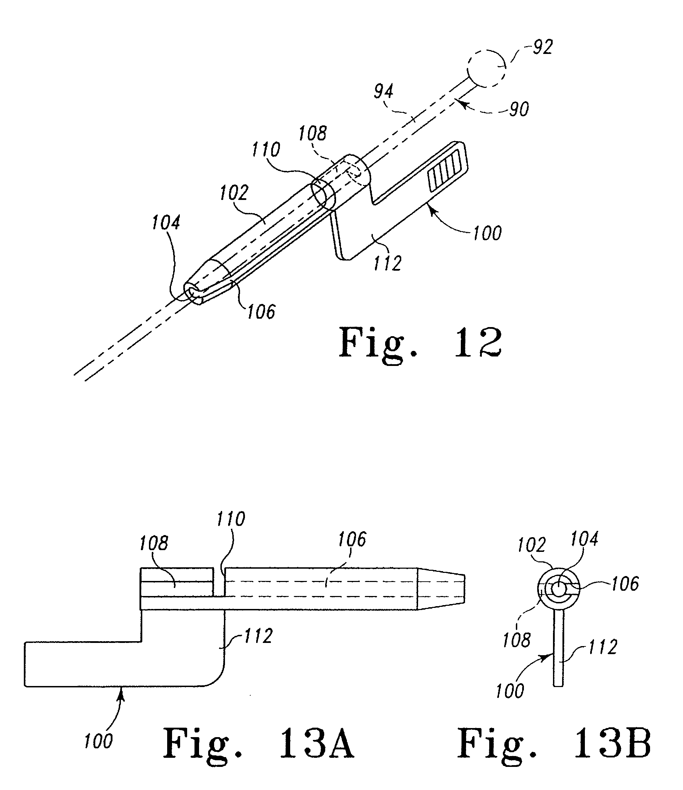

FIG. 12 is a perspective view of an exchange tool for use in a surgical procedure to fix the fixation system shown in FIG. 1 to a bone.
FIGS. 13a, 13b are side and end views of the exchange tool shown in FIG. 12.

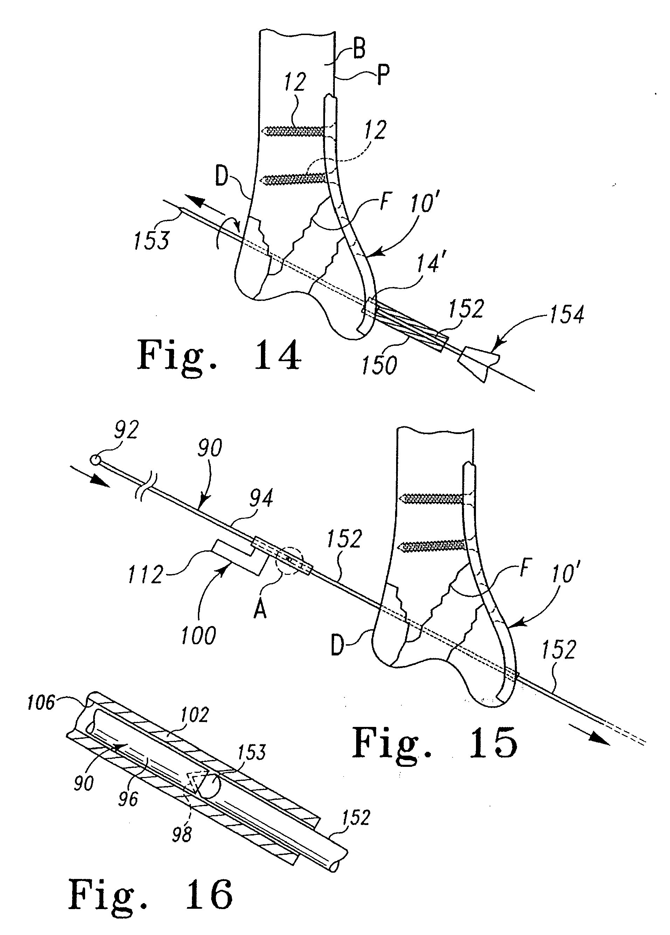

FIG. 14 is a representation of a first step in a method for fixing a fixation system as shown in FIG. 1 to reduce and fix a fractured bone, in accordance with one embodiment of the invention.
FIG. 15 is a representation of a further step in the method for fixing a fixation system to reduce and fix a fractured bone.
FIG. 16 is an enlarged partial cross-sectional view of the interface between a tension element and a K-wire, designated as area A in FIG. 15.

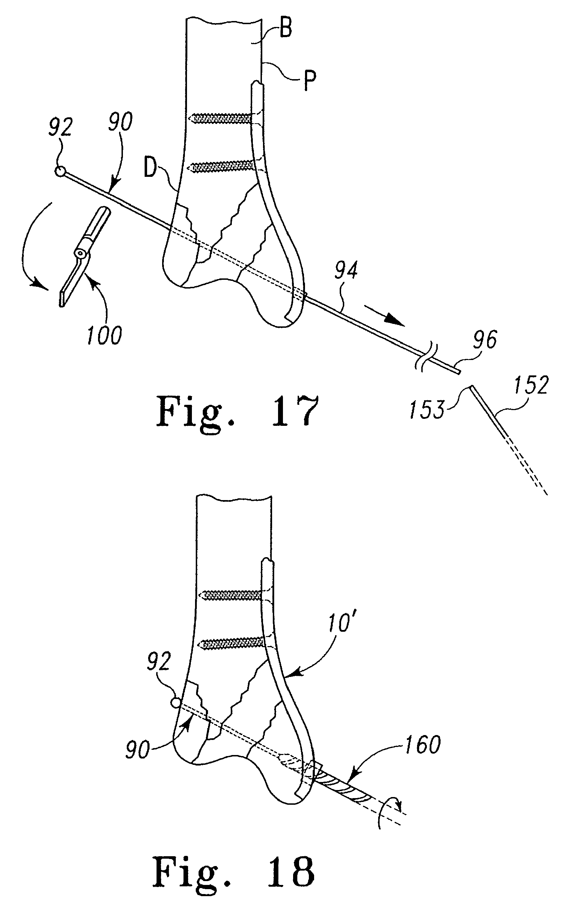

FIGS. 17-20 are representations of subsequent steps in the method for fixing a fixation system as shown in FIG. 1 in accordance with one embodiment of the invention.

Referring to the drawings, Figure 1 shows a system for reducing and fixing bone fractures, such as the fractures F in bone B. The present system can be used to treat fractures with multiple bone fragments that require precise reduction and secure fixation to maintain the reduction as the bone mends. In accordance with one embodiment, a bone plate 10 is positioned on the bone in a conventional manner. The bone plate may be of many known configurations that incorporate a series of recesses (FIG. 2). In one embodiment, bone screws 12 may be used to anchor one end of the bone plate 10 away from the fractures F. These screws help anchor the fixation arrangement in viable bone and can also help hold the plate as other fixation elements are introduced into the bone.

In accordance with the present invention, it is contemplated that a fixation system 20 includes a plurality of tension elements 22 that are adapted to work with the plate 10 to reduce the fractures F in the bone B. The tension elements are configured to extend through the recess 14 and opening 14a in the bone plate 10 and through a bore 18 formed in the bone, as shown in FIG. 2. The tension elements 22 are therefor sized to span from a proximal surface P to an opposite distal surface D of the bone B. The distal end of the tension elements 22 is provided with a distal anchor 24 which may come in a variety of forms that are adapted to anchor against the cortical bone C at the distal surface D of the bone when tension is applied to the tension element 22. A proximal anchor 26 engages the proximal end of the tension element to the bone plate at the proximal surface P of the bone and is configured to hold tension in the tension element 22.

The tension element 22 is an elongated flexible element that is capable of being pulled into tension and then anchored at its opposite ends. In certain embodiments, the tension element may be a braided metal cable, a monofilament or braided suture, or a biocompatible wire. When the fixation system 20 is initially installed, the tension element 22 includes an excess portion 28 that projects beyond the bone B and plate 10. Once the proximal anchor 26 is fixed, the excess portion 28 can be severed, leaving a cut end 29 that is preferably as close to the proximal surface P of the bone B as possible. The tension element can be severed using a tool appropriate for the particular material of the element.

In the embodiment illustrated in FIGS. 1 and 2, the proximal anchor 26 is a gripping washer, such as a Tinnerman washer. As is known in the art, a Tinnerman washer includes a conical central portion 26a having a central opening 26b. In accordance with the present invention, the central opening 26b has an effective diameter slightly less than the effective diameter of the tension element 22. As depicted in FIG. 2, the proximal anchor or washer 26 is positioned within the recess 14 in the plate 10 with the conical portion 26a projecting outward from the plate. With this orientation, the proximal anchor 26 can hold the tension in the element 22 since any pull opposite the tension direction T will tend to dig the element into the opening 26b and will try to compress the conical portion 26a. The walls of the recess 14 keep the washer 26 from flattening so that the washer will act to maintain the tension in the element 22 between distal and proximal anchors 24, 26.

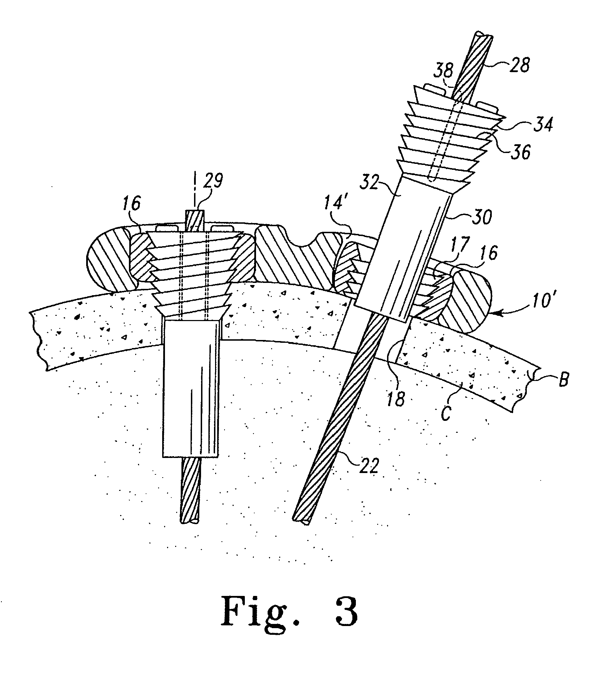

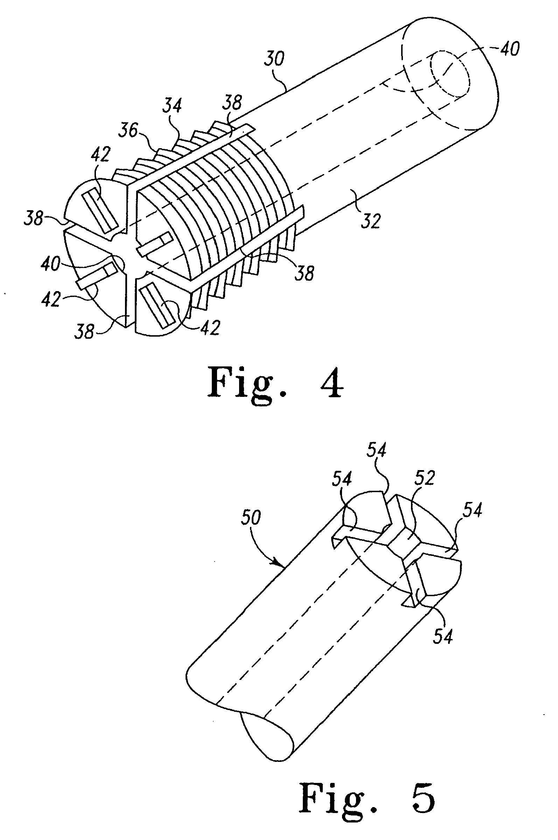

An alternative proximal anchor 30 is illustrated in FIGS. 3 and 4. This anchor is especially adapted for use with a bone plate 10' that is similar to the polyaxial locking plate disclosed in US-5954722. This locking plate includes a plurality of thru-holes 14' in which the wall of the holes is generally spherical. A locking bushing 16 similar to the bushing disclosed in US-5954722 is configured to fit within the spherical walls of the thru-holes 14' so that the bushing can assume a range of angular orientations relative to the plate 10'. The bushing 16 is provided with internal threads 17. As disclosed in US-5954722, the locking bushing is configured to receive the tapered threaded head of a bone screw so that as the head is threaded into the internal threads 17 the bushing 16 expands outwardly into the spherical surface of the thru-bore 14' to lock the bushing, and hence the bone screw, at the particular angular orientation relative to the locking plate.

The proximal anchor 30 of this embodiment includes a non-threaded shank 32 that is configured to extend through the plate 10' and into the bone bore 18 in the bone B. In one specific embodiment, the shank 32 may be tapered to form a press-fit engagement within the cortical bone C near the proximal surface P of the bone B. The proximal anchor 30 further includes a tapered head 34 that carries external threads 36 for engaging the internal threads 17 of the locking bushing 16. Like the bone screw in US-5954722, the tapered head 34 of the proximal anchor 30 expands the bushing as the head is threaded into the bushing, thereby locking the bushing within the spherical walls of the thru-hole 14' in the plate 10'. Thus, as with the bone screw disclosed in US-5954722, the proximal anchor 30 incorporates a polyaxial capability that allows the anchor to assume a range of angular orientations relative to the bone plate 10'. This polyaxial capability allows the tension element 22 passing through the anchor 30 to assume a comparable range of angles relative to the plate when the element is tensioned and anchored. Thus, it can be seen that the polyaxial capability enhances a surgeon's ability to reduce difficult bone fragments and hold that reduction over time.

As seen in FIG. 4, the proximal anchor 30 defines a central bore 40 through which the tension element 22 extends, as depicted in FIG. 22. In order to provide a clamping effect on the tension element, the anchor 30 further defines an array of cross-slots 38 passing through the central bore 40. As the head portion 34 is threaded into the locking bushing 16, the cross-slots 38 allow the effective diameter of the central bore 40 to decrease, thereby compressing about the tension element. As with the Tinnerman washer 26 discussed above, any pulling force on the tension element 22 will tend to pull the head 34 of the proximal anchor 30 deeper into the bushing 16, which has the effect of further closing the cross-slots and central bore around the tension element. Thus, the head portion 34 of the proximal anchor accomplishes two locking functions - first, locking the angular orientation of the anchor relative to the plate, and second, locking the tension element within the anchor.

In one specific embodiment, the head portion 34 defines driving lugs 42 on the proximal face thereof. A driving tool, such as the tool 50 shown in FIG. 5, may engage the lugs to permit rotation of the proximal anchor. The driving tool 50 is cannulated 52 with drive slots 54 configured to tightly engage the lugs 42 on the proximal anchor. The cannulated aspect of the tool 50 allows the tool to be used to drive the proximal anchor into the bone plate 10' while the excess portion 28 of the tension element is still available for tensioning the element.

In a method of using the fixation system 20 of the present invention, a bone plate 10, 10' is positioned on the bone B so that the plate openings 14a, 14' are optimally oriented for reducing the bone fragments of the fracture F. One end of the bone plate may be anchored to the bone B using fasteners, such as the screws 12 shown in FIG. 1. A drill guide is then mounted over a plate opening and an orthopaedic drill is used to form the bone bore 18 through the bone B, exiting at the distal surface D. A K-wire may be first inserted to help guide the bone drill. The bone bore 18 is preferably sized slightly larger than the effective diameter of the tension element 22. In the embodiment of the fixation system that uses the proximal anchors 30, a larger diameter bone bore 18 may be formed in the proximal cortical bone C for engagement by the shank 32 of the anchor, as depicted in FIG. 3.

Once the bone bore 18 has been formed, a tension element 22 is passed through the plate opening and bone bore until the distal anchor 24 has exited at the distal surface D of the bone. In certain embodiments, an introducer sheath (not shown) may be first positioned within the bone bore 18 to facilitate passage of the tension element. With the distal anchor 24 in position, the proximal anchor 26 or 30 is threaded onto the proximal end of the tension element. Tension may be applied to the tension element by pulling at the excess portion 28 of the element 22. A cable tensioning device, such as the device disclosed in US-6595994, may be adapted to engage the tension element 22 and apply tension to the element in a manner that permits fixation of the element by the proximal anchor. In one approach, the working end of the device disclosed in US-6595994 can bear against the proximal anchor Tinnerman washer 26 as the tension element or cable is pulled in the tension direction T (FIG. 2). In a further embodiment, the working end of the cable tensioning device disclosed in US-6595994 may be modified to bear against the proximal face of the anchor 30 (FIG. 4). Moreover, the working end of the tensioning device may be modified to incorporate the driving tool 50 (FIG. 5). In this further embodiment, once the appropriate tension has been applied to the element 22, the proximal anchor 30 may be tightened into the locking washer 16 to fix the construct.

The distal anchor 24 is configured to engage the distal surface D of the bone B when tension is applied and maintained in the tension element 22. In one specific embodiment, the distal anchor may include wings 25 (FIG. 2) that pivot outward once the distal end has exited the bone bore 18 at the distal surface D. The wings may be propelled outward by a torsion spring disposed within the distal tip of the tension element.

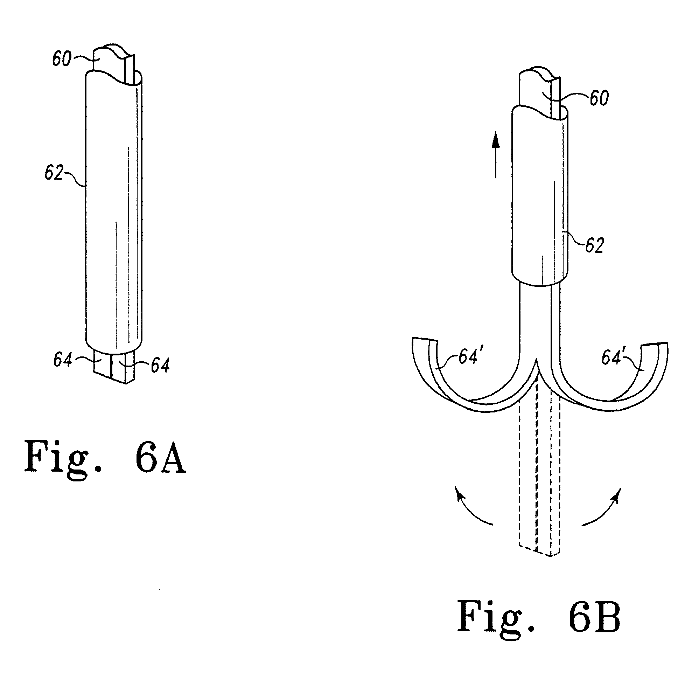

In an alternative embodiment illustrated in FIGS. 6a-7b, the distal anchor incorporates shape memory technology. In this embodiment, a tension element 60 includes prongs 64 that exhibit shape memory to move to an expanded orientation 64' shown in FIG. 6b. This shape memory feature may be provided by pre-bending the distal end of the tension element 60 so that the prongs spring outward to the position shown in FIG. 6b. In order to introduce the tension element through the plate 10/10' and the bone bore 18, the element 60 is threaded into an introducer sheath 62 with the prongs maintained within the sheath as the sheath and tension element are passed through the bone plate and bone bore. Once the prongs 64 are positioned at the distal surface D of the bone, the sheath may be retracted, allowing the prongs to assume their anchoring configuration. One benefit of this shape memory feature is that the tension element may be removed by dislodging the distal anchor, which can advantageously be accomplished by extending the sheath 62 back along the tension element until it contacts the prongs and draw them inward within the sheath.

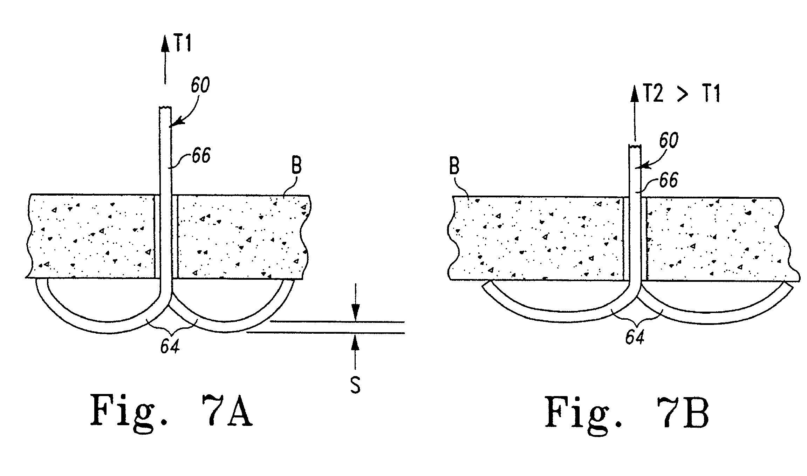

The tension element 60, and more particularly the shape memory prongs 64, may be configured to enhance their anchoring capability when the tension element 60 is tensioned. In particular, as illustrated in FIG. 7a, the tension element 60 may be positioned initially with the body 66 extending through the plate and bone and with the prongs 64 in an initial extended orientation. This initial orientation nominally corresponds to the free state of the shape memory prongs when left unconstrained. As tension is applied to the body 66, the prongs flatten from their initial orientation by a distance S. In this tensioned configuration the prongs 64 act as springs to increase the tension T2 in the body 66 from the original tension T1 (FIG. 7a). This characteristic of the tension element 60 may be used to control the amount of tension applied to the element and prevent over-tensioning. For instance, the "flatness" of the prongs may be gauged to provide an indication of the tension. It may also be contemplated that under excess tension the prongs 64 will invert and begin to move into the bone bore, which thus has the effect of dumping the tension in the element 60.

In one specific embodiment, the prongs 64 are formed of a spring material, such as medical grade spring steel. In another embodiment, the prongs are formed of a shape memory metal, especially a nickel-titanium alloy such as that referred to as Nitinol, which can change shape at a pre-determined temperature which can be arranged to be near to body temperature.

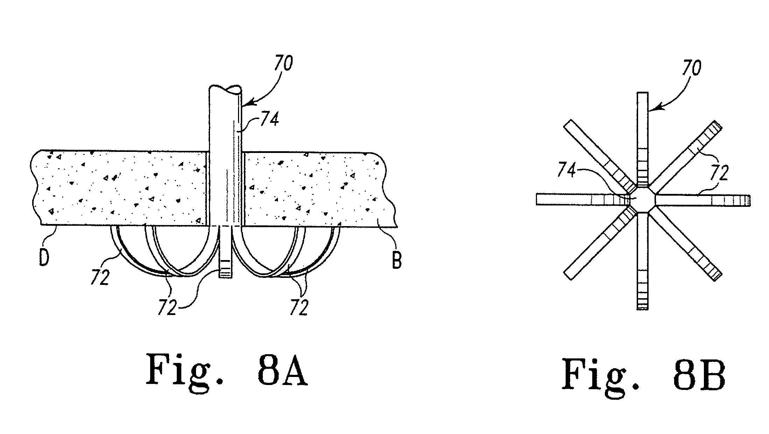

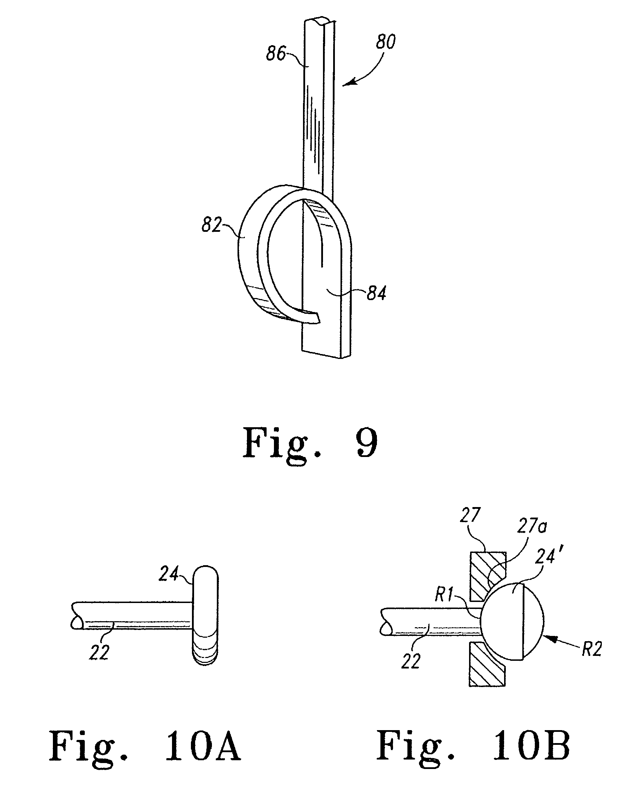

In the embodiment depicted in FIGS. 6a-6b, the tension element 60 includes a pair of opposite prongs 64. In another specific embodiment depicted in FIGS. 8a-8b, a tubular tension element 70 may include more than two prongs 72 extending from an elongated body 74. The multiple prongs 72 preferably exhibit the same shape memory characteristics as the prongs 64 described above. In yet another embodiment depicted in FIG. 9, a tension element 80 includes an elongated body 86 terminating in a distal portion 84. A curl element 82 separates from the distal portion 84 when the distal end is adjacent the distal surface D of the bone B. The curl element 82 may have a normal, unloaded radius of curvature, while the radius is reduced when tension is applied to the body 86. As the radius decreases, the curl element 82 exerts a greater resistance, thereby increasing the tension, as with the tension element 60 described above.

In other embodiments of the invention, the distal anchor may incorporate a fixed component. For instance, as shown in FIG. 10a, the tension element 22 terminates in a disc 24. In an alternative embodiment shown in FIG. 10b, the tension element 22 may terminate in a rounded element 24'. The rounded element 24' has one radius of curvature R1 and a larger radius of curvature R2 at the surface that contacts the soft tissue surrounding the bone in order to minimize the trauma to the adjacent tissue. The smaller radius R1 is configured to sit within a complementary configured recess 27a of a washer 27 that is disposed between the bone and the distal anchor. This washer/anchor interface permits variable angular orientations while ensuring uniform load distribution on the distal surface of the bone. In yet another specific embodiment, the distal anchor may constitute a spherical ball 92 fixed at the end of the body 94 of a tension element 90, as illustrated in FIG. 11a.

One common attribute of the embodiments shown in FIGS. 10a, 10b and 11a is that the distal anchors are, in effect, rigid, and incapable of passing through the plate opening 14/14' or the bone opening 18. Thus, unlike the shape memory anchors of FIGS. 6a-9, the tension elements with the distal anchors 24, 24' and 92 cannot be inserted from the proximal surface P of the bone B. A further embodiment of the invention contemplates a system and method for introducing these tension elements through the distal surface D to mate with a bone plate at the proximal surface P, all in a minimally invasive procedure.

In this embodiment, an exchange tool 100 that is used to facilitate retrograde movement of the proximal end 96 of the tension element 90 through a bone bore. As shown in FIGS. 12, 13a and 13b, the tool 100 includes a barrel 102 that defines a bore 104 through it that is sized to receive the elongated body 94 of the tension element 90. The barrel 102 defines a side opening 106 at a proximal portion of the barrel and a distal side opening 108 at a distal portion. The distal side opening 108 is offset from the proximal side opening 106 although the two side openings are contiguous through a connecting slot 110. The tool 100 further includes a handle 112 connected to the barrel 102 that is used to manipulate the exchange tool as described below. As best seen in FIG. 13b, the two side openings 106 and 108 are preferably about 180° apart.

The exchange tool is used in the series of steps depicted in FIGS. 14-19. In a first step, a bone plate 10' is shaped to fit a bone B adjacent a fracture site F. As described above, the bone plate 10' may be anchored by bone screws 12 positioned apart from the fracture site. A guide 150 is positioned within a plate opening 14' that is aligned with a desired fixation location. It can be appreciated that a pattern of fixation may be pre-planned based on radiographic images of the bone fragments. As shown in FIG. 14, a first tension element may be introduced to span numerous fractures at the head of the bone B. The guide 150 is sized to receive a K-wire 152. The K-wire is operatively coupled to a drill 154 that is used to drive the K-wire through the bone at a desired angle relative to the plate 10'. In the above-described embodiments that utilize the shape memory feature, it is necessary to form the bone bore 18 so that the distal anchor may be conveyed through the bone. In the embodiment of FIGS. 14-19, it is not necessary to initially form the bone bore since the distal anchor will be introduced from the distal surface D of the bone.

The K-wire 152 has a sharpened tip 153 adapted to penetrate bone. Other similar devices may be used, such as a thin gage stylet. In an alternative approach, a separate drill bit may be used to form a narrow bore through the bone, with the K-wire, stylet or similar elongated element advanced through the bore. In this alternative approach, the drilled bore preferably has a diameter smaller than the diameter of the K-wire or stylet.

Once the K-wire has exited the bone through the distal surface, the exchange tool 100 is positioned over the distal tip of the K-wire 152 so that the K-wire extends through the central bore 102, as shown in the detail view of FIG. 16. The proximal end 96 of the tension element 90 is then introduced into the bore 102 from the distal end of the tool 100. As shown in FIG. 11b, the proximal end 96 defines a conical recess 98. This recess is configured to accept the tapered tip 153 of the K-wire, as depicted in FIG. 16. It can thus be appreciated that the exchange tool 100 provides a mechanism for aligning the elongated body 94 of the tension element 90 with the K-wire 152 at the distal side of the bone B.

With the K-wire and tension element united within the exchange tool, the barrel 102 of the tool is preferably moved into contact with or at least immediately adjacent the distal surface D of the bone. The body 94 of the tension element 90 is then pushed retrograde toward the bone B, as shown in FIG. 15. As the tension element moves toward the bone, the proximal end 96 pushes the K-wire 152 out of the bone. It can be appreciated that once the K-wire/tension element interface reaches the bone B, the exchange tool 100 is no longer required to maintain that interface. Thus, the tool may be readily removed by rotating the tool in the direction of the arrow in FIG. 17 so that the tool essentially pivots about the connecting slot 110. As the tool is pivoted, the elongated body 94 of the tension element automatically exits the central bore 104 through the side slots 106, 108, leaving only a portion of the body 94 within the connecting slot 110. The exchange tool 100 is then completely removed by sliding the connecting slot 110 off the tension element.

With the exchange tool removed, the bore formed in the bone by the introduction of the K-wire serves to guide the tension element 90 toward the proximal surface P of the bone. Eventually the tip 153 of the K-wire exits the bone at the proximal surface P and the K-wire can be removed. The tension element is then advanced farther through the bone until the distal anchor 92 contacts the distal surface D of the bone. It can be appreciated that the exchange tool initially and the bore in the bone subsequently help maintain the tension element 90 in alignment. The central bore 104 in the exchange tool and the bone bore are only slightly greater in diameter than the body 94 of the tension element so the body cannot flex or buckle as the tension element is moved retrograde into and through the bone. Thus, even if the tension element exhibits lateral flexibility, it can still be advanced into the bone in the manner described.

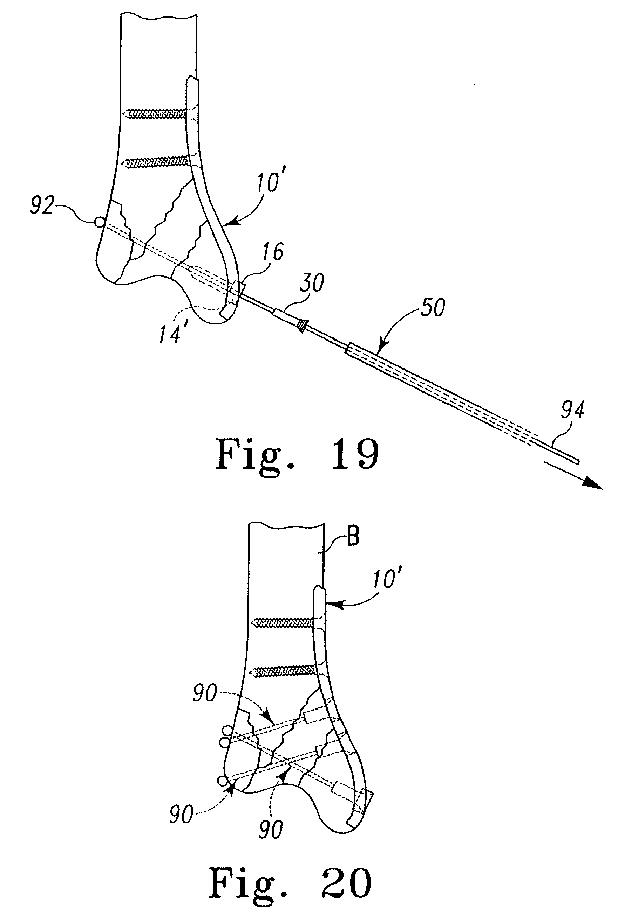

At this point, the tension element 90 is available to receive a proximal anchor to reduce the fracture and fix the tension element to the bone plate. In the case of the anchor 26 shown in FIGS. 1-2, the Tinnerman washer may be threaded onto the exposed portion of the tension element and tightened as described above. For the anchor 30 shown in FIGS. 3-4, the next step of the process includes advancing a cannulated drill 160 over the tension element, as depicted in FIG. 18. The cannulated drill may form the bone bore 18 to a depth sufficient to accept the shank 32 of the proximal anchor 30. This depth will vary depending upon the length of the shank 32. In some cases, the shank will extend across the bone to the cortical bone at the distal surface, while in the typical case the shank will only project partially into the bone. With tension maintained on the body 94 of the tension element, the proximal anchor 30 is driven into the bone using tool 50 and into locking bushing 16 of the plate 10', as illustrated in FIG. 19. This process can be repeated at a number of locations on the bone plate 10', as shown in FIG. 20. The tension at each tension element may be adjusted throughout the process, even as other tension elements are being added to the construct, to even out the plate compression on the bone or other wise adjust the position of the plate. Once the surgeon is satisfied with the tension and orientation of all of the tension elements, the excess tension element material may be removed.

The tension element 22 is preferably a braided metal cable or similar medical grade material suitable for implantation in a patient. In the preferred embodiment, the tension element does not elongate significantly under tension. It is expected that the tensioning process may be iterative as the tension element stretches slightly as load is applied. A gauge may be used to verify the cable tension. In alternative embodiments, the tension element is formed of a more elastic material, such as a silicone rubber cord, or may incorporate a spring element with a calibrated stiffness.

In the illustrated embodiments, a single tension element is provided at each hole location in the bone plate 10/10'. Alternatively, multiple tension elements may be anchored at a common plate recess 14 or opening 14'. Each tension elements may be deployed at different angles relative to the bone plate.

The tension elements and distal anchors may be provided in a range of sizes depending upon the type of fracture being treated. For instance, the tension element may have an effective diameter of about 1 to 3 mm and a length of about 100 to 200 mm. The distal anchors 24, 24' and 92 may have an effective diameter of about 4 to 6 mm.

The size of the proximal anchors are dictated in some degree by the dimensions of the bone plate to which these anchors are fixed. For instance, the diameter of the Tinnerman washer 26 or the threaded tapered head 34 of the anchor 30 are determined by the internal diameter of the recess 14 and locking bushing 16, respectively. The height of the proximal anchors are preferably set so that the anchors do not project outside the exposed surface of the bone plate, to minimize trauma to surrounding tissue.

In the certain embodiments, the distal anchors may be integral with the tension element. In other embodiments, the distal anchors are permanently attached to the tension element in a known manner, such as by crimping or welding. It is expected, however, that the attachment between tension element and distal anchor be sufficiently strong to withstand the tension applied to the element.

The proximal anchor 30 makes use of a tapered threaded engagement between the bone plate and the anchor, via the locking bushing. Alternatively, the opening 14' in the bone plate 10' (FIG. 3) may be tapered, such as a Morse taper. The threads 36 may be eliminated on the head 34 of the proximal anchor 30 (FIGS. 3-4) so that the head 34 is a smooth tapered component, while retaining the cross-slots 38. Rather than threading the proximal anchor into the locking bushing, the tapered head may be pressed into the mating tapered opening in the bone plate while the tension element 22 is maintained in tension. With this alternative embodiment, the driving lugs 42 may also be eliminated. The driving tool 50 may be replaced with a cannulated impact driver. In order to retain the polyaxial or variable angle capability, the locking bushing may incorporate the complementary mating angle for press-fit engagement with the tapered head of the modified proximal anchor. The plate opening 14' may then retain its spherical inner surface to mate with the spherical outer surface of the bushing at a range of angles relative to the plate.

## Claims

1. A bone fracture fixation system comprising:
a bone plate configured to bear against a proximal surface of the bone, the bone plate defining a plurality of openings through it;
an elongated tension element sized to pass through one of the plurality of openings and through the bone from the proximal surface to a distal surface thereof;
a distal anchor attached to the tension element and configured to engage the distal surface of the bone when the tension element passes through the bone; and
a proximal anchor which can be engaged between the bone plate and the tension element to maintain tension in the tension element between the proximal anchor and the distal anchor.

2. The bone fracture fixation system of claim 1, in which the proximal anchor has a central opening sized to engage the tension element passing through it.

3. The bone fracture fixation system of claim 1, in which the bone plate defines a recess around at least some of the plurality of openings; and the proximal anchor is sized to be received within the recess.

4. The bone fracture fixation system of claim 1, in which the proximal anchor is a Tinnerman washer.

5. The bone fracture fixation system of claim 1, in which the tension element is a metal cable.

6. The bone fracture fixation system of claim 1, in which the distal anchor has a shape memory component with a first configuration sized to pass through one of the plurality of openings and through the bone from the proximal surface to a distal surface thereof, and a second shape memory configuration for engaging the distal surface of the bone.

7. The bone fracture fixation system of claim 6, in which the distal anchor includes at least two prongs, the at least two prongs being substantially aligned with the elongated tension element in the first configuration and extended outward therefrom in the second shape memory configuration.

8. The bone fracture fixation system of claim 7, in which the prongs are spring elements.

9. The bone fracture fixation system of claim 6, in which the shape memory component is a strip configured to curl in the second shape memory configuration.

10. The bone fracture fixation system of claim 6, in which the distal anchor is formed of a shape memory metal.

11. The bone fracture fixation system of claim 1, in which the distal anchor is a disc.

12. The bone fracture fixation system of claim 1, in which the distal anchor is a generally spherical element.

13. The bone fracture fixation system of claim 12, in which the distal anchor includes a washer with a recess to receive the spherical element therein.

14. The bone fracture fixation system of claim 1, further comprising a sheath sized to contain the distal anchor and at least a portion of the tension element, the sheath adapted for insertion into the bone.

15. The bone fracture fixation system of claim 1, in which the proximal anchor is configured for a polyaxial interface with the bone plate.

16. The bone fracture fixation system of claim 15, in which the proximal anchor includes a shank extendable into the bone when the proximal anchor is engaged to the bone plate.

17. The bone fracture fixation system of claim 15, in which at least some of the openings define a spherical wall and include a locking bushing disposed therein, the locking bushing having a spherical outer surface for complementary engagement with the spherical wall, the locking bushing further including an internally threaded bore; and in which the proximal anchor includes a central bore for receiving the tension element therethrough and a threaded head, the head of the anchor and the locking bushing defining a tapered threaded interface so that the bushing expands into the spherical wall as the head is threaded into the bushing.
